# EUROPEAN PATENT APPLICATION

(11) **EP 2 757 798 A1**
(43) Date of publication of application: **23.07.2014**
(21) Application number: 13182477.3
(22) Date of filing: 30.08.2013
(51) Int. Cl.: H04N 21/442, H04N 21/45

(54) **Electronic device for determining emotion of user and method for determining emotion of user**

(30) Priority: 22.01.2013 KR 20130007049
(71) Applicant: Samsung Electronics Co., Ltd, Gyeonggi-do 443-742 (KR)
(72) Inventor: Kim, Kwang-soo, Gyeonggi-do (KR); Son, Chang-won, Daegu (KR); Jung, Do-sung, Seoul (KR)
(74) Representative: Walaski, Jan Filip

(57) **Abstract**

A system and method for determining an emotional state of a user of an electronic device. The system is configured to execute a method of receiving, using the electronic device, input emotional data, comparing the input emotional data with reference emotional data and determining an emotional state of a user of the electronic device, and transmitting the determined emotional state of the user to an external device to share the emotional state with the external device.

## Description

The present invention relates to an electronic device for determining a user's emotion and a method thereof for determining a user's emotion, and more particularly, to an electronic apparatus and a method for determining a user's emotion and sharing the determined emotion with other electronic devices based on text or voice data input by a user.

Recent electronic devices, such as a TV, have attempted to identify a user's emotions.

Such conventional technology for identifying a user's emotions requires a user to directly input their emotional state to the electronic device. For example, a TV asks a user about his/her emotional state through a pop message, etc. and a user expresses his/her emotion through a voice recognition technology. Also, a user's physical state may be checked using a motion capture of a Multimedia over Internal Protocol (MoIP) camera, and a heat sensor.

However, this conventional method for identifying the user's emotions requires a sophisticated input of the emotional state by a user and the physical checking method results in uncertainty of the user's emotional state. In particular, due to the sophisticated input for identifying emotion of the user, a sufficiently sized database cannot be established, and thus the conventional method cannot be widely used.

Further, electronic devices such as a display apparatus or air conditioner may not easily identify the emotional state of the user due to limited user input means.

Accordingly, one or more exemplary embodiments provide an electronic device and a method for determining emotion of a user using data input during a typical usage process of the electronic device without directly receiving a user's input of emotional state.

Another exemplary embodiment provides an electronic device for extracting emotion of a user through a mobile terminal such as a mobile phone that is most frequently used to express the user's emotion and for sharing the extracted emotional state with other electronic devices.

Still another exemplary embodiment provides a cloud-based system and a method which extracts emotion of a user from various types of electronic devices and includes a database of the extracted emotional state data.

Yet another exemplary embodiment provides an electronic device for identifying emotion of a user, analyzing a reaction pattern according to emotion and providing service suitable for emotion of the user.

Yet another exemplary embodiment provides an electronic device and a method for discriminatively collecting extracted emotional data with respect to various users and determining emotion of each user by using the collected data.

According to an aspect of an exemplary embodiment, there is provided a method for determining an emotional state of a user of an electronic device including, receiving, using the electronic device, input emotional data, comparing the input emotional data with reference emotional data and determining an emotional state of a user of the electronic device, and transmitting the determined emotional state of the user to an external device to share the emotional state with the external device.

The reference emotional data may be stored at least one of in a storage unit and a cloud server connected through communication.

The input emotional data may be obtained from voice data input by the user.

The input emotional data may be obtained from text data input by the user.

The method for determining an emotional state of a user of an electronic device may further include correlating the determined emotional state with at least one of intensity of the input voice data, speed of the input voice data, and rate of recognition of the input voice data, and storing the correlated emotional state.

The method for determining an emotional state of a user of an electronic device may further include correlating the determined emotional state with least one of an input speed of the input text data, rate of typographical error of the input text data, and intensity of touching a touch screen when inputting the text data, and storing the correlated emotional state.

The method for determining an emotional state of a user of an electronic device may further include correlating the determined emotional state with at least one of motion history of a functional control operation, a predetermined application executing operation, and a predetermined content outputting operation of the electronic device as a reaction operation according to the emotional state of the user for predetermined time after determining the emotional state of the user, and registering the correlated emotional state.

The method for determining an emotional state of a user of an electronic device may further include in response to the emotional state of the user being determined, recommending to the user at least one of a function of the electronic device, an execution of an application, and output of content, corresponding to the emotional state based on the registering.

The method for determining an emotional state of a user of an electronic device may further include storing the determined emotional state of the user in at least one of a storage unit and a cloud server connected through a communication network.

The motion history correlated with the emotional state may be recorded in at least one of a storage unit and a cloud server connected through a communication network.

The motion history correlated with the emotional state may be transmitted to an external device together with the emotional state of the user.

According to an aspect of another exemplary embodiment, there is provided a method for controlling an electronic device according to an emotional state of a user including, receiving, using the electronic device, emotional state data of a user that is determined using an external device connected through a communication network, checking a reaction record of the user corresponding to the emotional state of the user transmitted by the external device, and controlling the electronic device according to the checked reaction of the user.

The reaction record may include a reaction record according to the emotional state of the user stored in the external device.

The emotional state data of a user determined using the external device and the reaction record according to the emotional state in the external device may be transmitted through a cloud server connected to the communication network.

The method for controlling an electronic device according to an emotional state of a user may further include receiving input emotional data from the user of the electronic device, and comparing the input emotional data with reference emotional data and determining the emotional state of the user.

The method for controlling an electronic device according to an emotional state of a user may further include transmitting the determined emotional state of the user to the external device to share the emotional state with the external device.

The determined emotional state data of the user may be stored in at least one of a storage unit and a cloud server connected through a communication network.

The method for controlling an electronic device according to an emotional state of a user may further include comparing the emotional state of the user transmitted by the external device with a previous emotional state, recommending, if the compared emotional states matching, an application program or content which is in the same genre or category as that of the application program or content which was recommended for the previous emotional state, and recommending, if the compared emotional states are different, an application program or content in the same genre or category as that of an application program or content falling under a new emotional state.

According to an aspect of another exemplary embodiment, there is provided an electronic device for determining an emotional state of a user including, a user input unit configured to receive input emotional data, an emotional determiner configured to compare the received input emotional data with reference emotional data and to determine the emotional state of the user, and a communication unit configured to transmit the determined emotional state of the user.

The electronic device for determining an emotional state of a user may further include a storage unit configured to store the reference emotional data.

The reference emotional data may be stored in a cloud server connected through a communication network.

The input emotional data may be obtained from voice data input by the user.

The storage unit may store the determined emotional state by correlating the determined emotional state with at least one of intensity, speed, and rate of recognition of the voice data input by the user.

The input emotional data may be obtained from voice data input by the user.

The cloud server may store the determined emotional state by correlating the determined emotional state with at least one of intensity, speed, and rate of recognition of a voice data input by the user.

The input emotional data may be obtained from text data input by the user.

The storage unit may store therein the determined emotional state by correlating the determined emotional state with at least one of input speed, rate of typographical error of a text data input by the user, and intensity of touching a touch screen.

The input emotional data may be obtained from text data input by the user.

The cloud server may store therein the determined emotional state by correlating the determined emotional state with at least one of input speed, rate of typographical error of a text data input by the user, and intensity of touching a touch screen.

The storage unit may include the determined emotional state data of the user.

The cloud server may include the determined emotional state data of the user.

The storage unit may store the determined emotional state by correlating the emotional state with at least one of motion history of a functional control operation, a predetermined application executing operation, and a predetermined content outputting operation of the electronic device as a reaction operation according to the emotional state of the user for predetermined time after determining the emotional state.

The cloud server may store the determined emotional state by correlating the emotional state with at least one motion history of a functional control operation, a predetermined application executing operation and a predetermined content outputting operation of the electronic device as a reaction operation according to the emotional state of a user for predetermined time after determining the emotional state.

The electronic device for determining an emotional state of a user may further include a user interface (UI) generator configured to generate, in response to the emotional state of the user being determined, a UI for recommending to the user at least one of a function of the electronic device, execution of an application, and an output of a content corresponding to the emotional state based on the stored reaction motion.

According to an aspect of another exemplary embodiment, there is provided an electronic device for determining an emotional state of a user including, a communication unit configured to receive emotional state data of a user that is determined using an external device, and a controller configured to check a reaction record of the user corresponding to the emotional state of the user transmitted by the external device and to control the electronic device according to the checked reaction of the user.

The reaction record may include a reaction record according to the emotional state of the user from the external device.

The emotional state data of a user extracted by the external device and the reaction record according to the emotional state from the external device are transmitted by a cloud server.

The electronic device for determining an emotional state of a user may further include a user input unit configured to receive input emotional data from the user of the electronic device, and a user emotional determiner configured to compare the input emotional data with reference emotional data and to determine the emotional state of the user.

The communication unit may transmit the determined emotional state of the user to the external device to share the emotional state with the external device.

The electronic device for determining an emotional state of a user may further include a storage unit configured to store the determined emotional state data of the user.

The determined emotional state data of a user may be stored in a cloud server connected through a communication network.

The controller may compare the emotional state of the user transmitted by the external device with a previous emotional state, recommend, in response to the compared emotional states matching, an application program or content in the same genre or category as that of the application program or content recommended for the previous emotional state, and recommend, in response to the compared emotional states being different, an application program or content in the same genre or category as that of the application program or content falling under a new emotional state.

According to an aspect of another exemplary embodiment, there is provided a method for controlling an electronic device by sharing an emotional state of a user including, receiving, using a first electronic device, input emotional data and determining an emotional state of a user based on the input emotional data, transmitting the determined emotional state of the user to a second electronic device to share the determined emotional state of the user with the second device, receiving the emotional state data of the user determined using the first electronic device connected to the second electronic device through a communication network, and checking a reaction record of the user corresponding to the emotional state of the user transmitted by the external device and controlling the second device according to the checked reaction of the user.

The reaction record may include a reaction record according to the emotional state of the user stored in the first electronic device.

The emotional state data of a user extracted by the first electronic device and the reaction record according to the emotional state in the first electronic device are transmitted by a cloud server.

The method for controlling an electronic device by sharing an emotional state of a user may further include receiving, using the second electronic device, input emotional data from the user input unit, and determining the emotional state of the user based on the input emotional data of the second electronic device.

The method for controlling an electronic device by sharing an emotional state of a user may further include transmitting the emotional state of the user determined using the second electronic device to the first electronic device to share the emotional state of the user with the first electronic device.

The emotional state data of a user determined by the first electronic device may be stored in at least one of a first storage unit of the first electronic device and a cloud server connected through the communication network.

The emotional state data of a user determined using the second electronic device may be stored in at least one of a second storage unit of the second electronic device and a cloud server connected through the communication network.

The method for controlling an electronic device by sharing an emotional state of a user may further include comparing, using the second electronic device, the emotional state of the user transmitted by the first electronic device and a previous emotional state, recommending, in response to the compared emotional states matching, an application program or content in the same genre or category as that of the application program or content recommended for the previous emotional state, and recommending, in response to the compared emotional states being different, an application program or content in the same genre or category as that of an application program or content falling under a new emotional state.

According to an aspect of another exemplary embodiment, there is provided a system for sharing an emotional state of a user between electronic devices including, a first electronic device including: a first user input unit configured to receive input emotional data from a user, a first emotional determiner configured to compare the received input emotional data with reference emotional data and to determine an emotional state of the user, and a first communication unit configured to transmit the determined emotional state of the user, and a second electronic device including: a second communication unit configured to receive emotional state data of the user determined using the first electronic device, and a controller configured to check a reaction record of the user corresponding to the received emotional state of the user from the first electronic device and to perform a control operation according to the checked reaction of the user.

The reaction record may include a reaction record according to the emotional state of the user received from the first electronic device.

The second electronic device may further include a second user input unit configured to input emotional data, and a second emotional determiner configured to determine the emotional state of the user based on the input emotional data that is input through the second user input unit.

The system for sharing an emotional state of a user between electronic devices may further include a second communication unit configured to transmit the emotional state of the user determined using the second electronic device to the first electronic device to share the determined emotional state with the first electronic device.

The first and second electronic devices include a first storage unit and a second storage unit, respectively, and the first storage unit is configured to store at least one of the emotional state data of the user determined using the first electronic device, the reference emotional data, and the reaction record according to the emotional state in the first electronic device, and the second storage unit is configured to store the emotional state data of the user determined using the first electronic device, the reference emotional data, the reaction record according to the emotional state in the first electronic device, emotional state data of the user determined using the second electronic device and the reaction record according to the emotional state in the second electronic device.

The system for sharing an emotional state of a user between electronic devices may further include a cloud server configured to be interposed between the first and second electronic devices, wherein the cloud server is configured to store the emotional state data of the user determined using the first electronic device, the reference emotional data, the reaction record according to the emotional state in the first electronic device, the emotional state data of the user determined using the second electronic device and the reaction record according to the emotional state in the second electronic device and to transmit the aforementioned data and records to the first and second electronic devices.

The second electronic device may further include an emotional comparer configured to compare the emotional state of a user transmitted by the first electronic device with a previous emotional state, and a user interface (UI) generator configured to generate, in response to the compared emotional states matching, a UI for recommending an application program or content in the same genre or category as that of the application program or content recommended for the previous emotional state, and to generate, in response to the compared emotional states being different, a UI for recommending an application program or content in the same genre or category as that of an application program or content falling under a new emotional state.

According to an aspect of another exemplary embodiment, there is provided a method for determining an emotional state of a user, including receiving, using a first electronic device, input emotional data, comparing the input emotional data with reference emotional data and determining an emotional state of the user, and transmitting the determined emotional state of the user to an external device to cause the external device to perform an action according to the determined emotional state.

The above and other aspects will become apparent and more readily appreciated from the following description of the exemplary embodiments, taken in conjunction with the accompanying drawings, in which:
FIG. 1 illustrates a process for sharing a determined emotional state of a user between a mobile terminal and a TV according to an exemplary embodiment;
FIG. 2 illustrates a process for sharing a determined emotional state of a user on a cloud service basis in which a server is provided between a mobile terminal and a TV according to another exemplary embodiment;
FIG. 3 is a block diagram of an electronic device for determining an emotion of a user according to an exemplary embodiment;
FIG. 4 is a flowchart showing a method for determining an emotion of a user of the electronic device according to an exemplary embodiment; and
FIG. 5 is a flowchart showing a method for extracting an emotion of a user of the electronic device according to another exemplary embodiment.

Below, exemplary embodiments will be described in detail with reference to accompanying drawings so as to be easily realized by a person having ordinary knowledge in the art. The exemplary embodiments may be embodied in various forms without being limited to the exemplary embodiments set forth herein. Descriptions of well-known parts are omitted for clarity, and like reference numerals refer to like elements throughout.

Determining an emotion of a user using a first electronic device 100 according to an exemplary embodiment may be achieved using an emotion determining application installed in a mobile terminal such as a smart phone. Of course, the first electronic device 100 may employ any electronic device including but not limited to a personal digital assistant (PDA), a laptop computer, a navigation device, a digital TV, a desktop computer, etc. as long as the electronic device has the same emotion determining application installed therein and may receive input emotional data such as text or voice.

Even electronic devices, to which input of emotional data is limited, such as an air conditioner or refrigerator, may be controlled according to the emotional state of the user by sharing the emotional state through a cloud server or another electronic device for which it is easy to input the emotional data.

Hereinafter, it will be explained for convenience that an emotion of a user is determined using a mobile terminal and the determined emotional state of the user is transmitted to a digital TV for sharing. Of course, the digital TV may determine and transmit the emotion of the user to the mobile terminal. However, it is more preferable to determine the emotion of the user using the mobile terminal, which is configured to allow an emotion of the user to be input. The emotion of the user may be input in various manners such as, for example, social networking service (SNS) including Facebook and Twitter, a mobile messenger (MIM), multimedia messaging service (MMS), voice call, video call, etc.

Between different electronic devices sharing the emotional state of the user, "user" means a single user using the different electronic devices, and each electronic device may include a means for identifying the user.

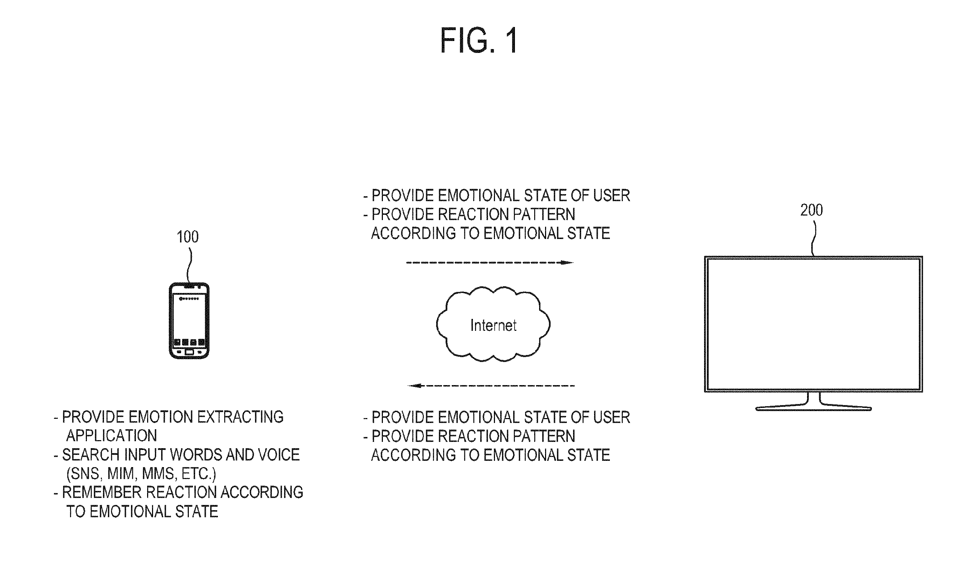

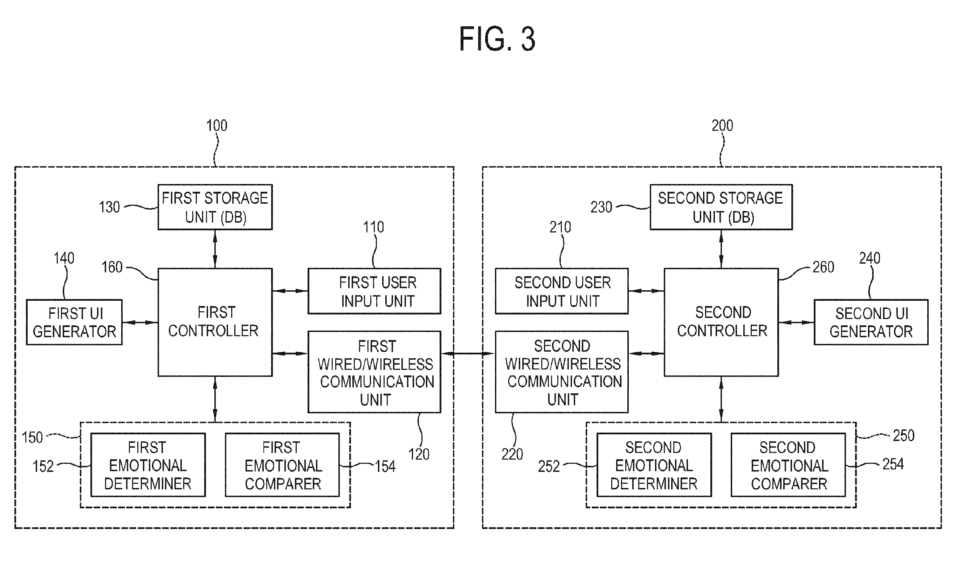

Referring to Fig. 1, the first electronic device 100 has a function for determining an emotion of a user according to an exemplary embodiment. As shown in Fig. 3, the first electronic device 100 may include a first storage unit (DB) 130 which classifies types of human emotions, and matches the classified emotion and reference emotional data for storage, a first user input unit 110 which receives input emotional data from the user, and a first emotional determiner 150 which compares the received input emotional data with the reference emotional data stored in the first storage unit 130 and determines the emotional state of the user.

Emotions of the user may be classified into different types. By way of example, the emotions of the user may be classified into four types such as pleasure, anger, sadness and happiness, and may be further classified as necessary. That is, the present disclosure is not limited to the above-mentioned four types of emotions and more or less types of emotion classifications can be used. For convenience, four types of emotions will be explained as an example.

The first storage unit 130 may match the four types of emotions and a number of texts and voice data (hereinafter, to be called "reference emotional data") and store the emotions.

The reference emotional data including the text or sentence may be determined from words or sentences falling under emotional expression out of texts input by a user in, e.g., SNS such as Facebook and Twitter, a mobile messenger (MIM), and multimedia messaging service (MMS). The reference emotional data including texts may include various types of emotions, e.g., such as basic words or sentences including "pleased", "excited", "angry", "annoying", "sad" and "happy", and adjectives, adverbs, exclamations, slangs, etc.

The first storage unit 130 may correlate at least one of input speed, and rate of typographical error of the input text and intensity of touching a touch screen with the determined emotional state and store the correlated emotional state.

The reference emotional data including voice may be stored as voice data correlated to the classified emotions. Preferably, the voice data input through the first user input unit 110 may be processed to recognize voice, identify the speech of the user, and determine a basic sentence therefrom, and the basic sentence may match with the classified emotions to be stored.

The first storage unit 130 may correlate at least one of intensity, speed, and rate of recognition of the input voice with the determined emotional state and store the correlated emotional state.

If the emotional state of the user is determined, the first storage unit 130 may correlate at least one motion history of a functional control operation, a predetermined application executing operation and a predetermined content outputting operation of the electronic device as a reaction operation according to the emotional state of the user for predetermined time and store the correlated emotional state.

The first storage unit 130 may temporarily store therein processing and controlling programs for the first controller 160 and input/output data.

The first storage unit 130 may store therein input emotional data input through the first user input unit 110 or first wired/wireless communication unit 120, and emotional state data of the user determined by the external electronic device 200 transmitted through the first wired/wireless communication unit 120.

The first storage unit 130 may include at least one storage medium of a flash memory type, hard disk type, multimedia card micro type, a card-type memory (e.g., SD or XD memory), random access memory (RAM), static random access memory (SRAM), read only memory (ROM), electrically erasable programmable read-only memory (EEPROM), programmable read-only memory (PROM), a magnetic memory, magnetic disk, optical disk, etc.

The first user input unit 120 may include one or more of a microphone, a keyboard, a mouse, a remote controller, and a touch panel.

The first emotional determiner 150 may be embodied as a program for determining the user's emotion.

For example, the first emotional determiner 150 may include a first emotional determiner 152 and a first emotional comparer 154.

The first emotional determiner is configured to compare the reference emotional data using the emotional state stored in the first storage unit 130 and the input emotional data input by the first user input unit 110 or from the outside and to determine the emotion of the user. If the input emotional data and the reference emotional data stored in the first storage unit 130 do not match, the emotion of the user may not be determined or the input emotional data of the first storage unit 130 may be registered as the reference emotional data to extend the database.

The first emotional comparer 154 is configured to compare the emotional state of the user stored in the first storage unit 130 and the emotional state measured by another electronic device input through the first wired/wireless communication unit 120. According to the identified emotional state, the first emotional comparer 154 may select at least one of reaction operations, e.g., a functional control operation, a predetermined application executing operation, and a predetermined content outputting operation of the electronic device. The selected reaction operation may recommend a reaction according to the emotional state of the user.

The first electronic device 100 which may determine emotions according to the exemplary embodiment may further include a first user interface (UI) generator 140 that generates a UI for recommending to the user at least one of the function of the electronic device corresponding to the emotional state, the application program executing operation and the content outputting operation based on the record of the emotional state if the emotional state is determined. The first electronic device may further include the first wired/wireless communication unit 120 that communicates with the other electronic device 200, and a first controller 160 which controls overall elements of the first electronic device 100.

The first UI generator 140 may generate a UI such as a message or pop-up window displayed in a screen (not shown). The user may input a command through the UI displayed in the screen. The UI may include a recommending or encouraging message or a pop-up window for selection based on the reaction according to the emotional state of the user. For example, if it is stored that the user is angry and listens to a particular music with a volume raised, the emotional state of the user may be determined to be angry and a pop-up window recommending the user the music for the concerned emotional state may be displayed.

The first controller 160 may control each element of the first electronic device 100. For example, the first controller 160 may control the first wired/wireless communication unit 120 to exchange the emotional state data of the user with other electronic devices, or store data in, or read data from, the first storage unit 130 or control the first emotional determiner 150 to compare the data input through the first user input unit 110 and the data stored in the first storage unit 130 for determination.

The first controller 160 may include Application Specific Integrated Circuits (ASICs), Digital Signal Processors (DSPs), Digital Signal Processing Devices (DSPDs), Programmable Logic Devices (PLDs), Field Programmable Gate Arrays (FPGAs), processors, controllers, micro-controllers, and microprocessors.

The first wired/wireless communication unit 120 may receive emotional state data of the user that has been determined by another electronic device 200, input emotional data input by the another electronic device 200, and reaction history according to the emotional state. The first wired/wireless communication unit 120 of the electronic device 100 may also transmit data including the emotional state data determined by the electronic device 100, input emotional data input to the electronic device 100, a reaction history according to the emotional state, etc.

The first wired/wireless communication unit 120 may employ data communication such as Very High-Data Rate Digital Subscriber Line (VDSL), Ethernet, Token Ring, high definition multimedia interface (HDMI), universal serial bus (USB), low voltage differential signaling (LVDS), and HDMI Ethernet Channel (HEC); mobile communication such as 2G, 3G, 4G and Long Term Evolution (LTE); wireless Internet technology such as Wireless LAN (WLAN) (Wi-Fi), Wireless broadband (Wibro), World Interoperability for Microwave Access (Wimax), and high speed downlink packet access (HSDPA); and local area communication technology such as Bluetooth, radio frequency identification (RFID), infrared Data Association (IrDA), Ultra Wideband (UWB), and ZigBee.

The configuration of the first electronic device 100 has been explained as above. The first electronic device 100 with the foregoing configuration may share data relating to emotions of the user with the second electronic device, e.g., a digital TV 200.

The second electronic device 200 is configured to communicate and share emotional data with the first electronic device 100. As shown in FIG. 3, the second electronic device 200 may include a second wired/wireless communication unit 220, a second storage unit (DB) 230, a second user input unit 210, and a second emotional determiner 250. The second wired/wireless communication unit 220 is configured to exchange data relating to emotion of the user with the first electronic device 100. The second storage unit (DB) 230 is configured to classify types of human emotions and match the classified emotion with reference emotional data. The second user input unit 210 is configured to receive the input emotional data from the user. The second emotional determiner 250 is configured to compare the received input emotional data with the reference emotional data stored in the second storage unit 230 and determine the emotional state of the user.

The second wired/wireless communication unit 220 may, directly or through the cloud server 300, receive data relating to the emotion of the user from the first electronic device 100, e.g., input emotional data of the user input using the first electronic device 100, reference emotional data of the user, the emotional state data of the user determined using the first electronic device 100, or reaction data according to the emotion of the user in the first electronic device 100. Also, the second wired/wireless communication unit 220 may, directly or through the cloud server 300, transmit the input emotional data of the user input using the second electronic device 200, reference emotional data of the user, emotional state data of the user determined using the second electronic device 200, and reaction data according to the emotion of the user in the second electronic device 200.

The second wired/wireless communication unit 220 may employ data communication such as Very High-Data Rate Digital Subscriber Line (VDSL), Ethernet, Token Ring, high definition multimedia interface (HDMI), universal serial bus (USB), low voltage differential signaling (LVDS), and HDMI Ethernet Channel (HEC); mobile communication such as 2G, 3G, 4G and Long Term Evolution (LTE); wireless Internet technology such as Wireless LAN (WLAN) (Wi-Fi), Wireless broadband (Wibro), World Interoperability for Microwave Access (Wimax), and high speed downlink packet access (HSDPA); and local area communication technology such as Bluetooth, radio frequency identification (RFID), infrared Data Association (IrDA), Ultra Wideband (UWB), and ZigBee.

The second storage unit 230 is configured to match the four types of emotions with a number of texts and voice data (hereinafter, to be called "reference emotional data") and store the emotions.

The reference emotional data including the text or sentence may be determined from words or sentences falling under emotional expression out of texts input by a user in, e.g. SNS such as Facebook and Twitter, MIM, and MMS. The reference emotional data including texts may include various types of emotions, e.g., such as basic words or sentences including "pleased", "excited", "angry", "annoying", "sad" and "happy", and adjectives, adverbs, exclamations, slangs, etc.

The second storage unit 230 is configured to correlate at least one of input speed, and rate of typographical error of the input text and intensity of touching a touch screen with the determined emotional state and store the correlated emotional state.

The reference emotional data including voice may be stored as voice data correlated with the classified emotions. Preferably, the voice data input through the second user input unit 210 may be processed to recognize a voice, identify the speech of the user, and determine a basic sentence therefrom, and the basic sentence may match with the classified emotions to be stored.

The second storage unit 230 is configured to correlate the determined emotional state with at least one of intensity, speed, and rate of recognition of the input voice and store the correlated emotional state.

If the emotional state of the user is determined, the second storage unit 230 may correlate the determined emotional state to at least one of motion history of a functional control operation, a predetermined application executing operation, and a predetermined content outputting operation of the electronic device as a reaction operation according to the emotional state of the user for predetermined time and store the correlated emotional state.

The second storage unit 230 may temporarily store therein processing and controlling programs for the second controller 260 and input/output data.

The second storage unit 230 may store therein input emotional data input of the first electronic device 100 or the cloud server 300 input through the second user input unit 210 or second wired/wireless communication unit 220, and emotional state data of the user determined using the first electronic device 100 or cloud server 300 transmitted through the second wired/wireless communication unit 220.

The second storage unit 230 may include at least one storage medium of a flash memory type, hard disk type, multimedia card micro type, a card-type memory (e.g., SD or XD memory), random access memory (RAM), static random access memory (SRAM), read only memory (ROM), electrically erasable programmable read-only memory (EEPROM), programmable read-only memory (PROM), a magnetic memory, magnetic disk, optical disk, etc.

The second user input unit 220 may include at least one of a microphone, a keyboard, a mouse, a remote controller, a touch panel, etc.

The second emotional determiner 250 may be embodied as a program for determining emotion of the user.

For example, the second emotional determiner 250 may include a second emotional determiner 252. The second determiner is configured to compare the reference emotional data using an emotion stored in the second storage unit 230 with the input emotional data input using the second user input unit 210 or from the outside and to determine the emotion of the user. If the input emotional data and the reference emotional data stored in the second storage unit 230 do not match, the emotion of the user may not be determined or the input emotional data of the second storage unit 230 may be registered as the reference emotional data to extend the database.

The second emotional determiner 250 may include a second emotional comparer 254 that is configured to compare the emotional state of the user stored in the second storage unit 230 and the emotional state measured using another electronic device 100 input through the second wired/wireless communication unit 220. According to the identified matching emotional state, the second emotional comparer 254 may select at least one of reaction operations, e.g., a functional control operation, a predetermined application executing operation, and a predetermined content outputting operation of the electronic device. The selected reaction may recommend a reaction according to the emotional state of the user.

The second electronic device 200 is configured to share the emotional state of the user with the first electronic device 100 according to the exemplary embodiment may include a second user interface (UI) generator 240 that is configured to generate a UI for recommending a user at least one of the function of the electronic device corresponding to the emotional state, the application program executing operation and the content outputting operation based on the record of the emotional state if the emotional state is determined. The second electronic device 200 may also include a second controller 260 that is configured to control overall elements of the second electronic device 200.

The second UI generator 240 is configured to generate a UI such as a message or pop-up window displayed in a screen (not shown). The user may input a command through the UI displayed in the screen. The UI may include a recommending or encouraging message or a pop-up window for selection based on the reaction according to the emotional state of the user. For example, if it is stored that the user is angry and listens to a particular music with a volume raised, the emotional state of the user may be determined to be angry and a pop-up window recommending the user the music for the concerned emotional state may be displayed.

The second controller 260 may control each element of the second electronic device 200. For example, the second controller 260 may control the second wired/wireless communication unit 220 to exchange the emotional state data of the user with the first electronic device 100, or store data in, or read data from, the second storage unit 230 or control the second emotional determiner 250 to compare the data input through the second user input unit 210 and the data stored in the second storage unit 230 or cloud server 300 for determination.

The second controller 260 may include Application Specific Integrated Circuits (ASICs), Digital Signal Processors (DSPs), Digital Signal Processing Devices (DSPDs), Programmable Logic Devices (PLDs), Field Programmable Gate Arrays (FPGAs), processors, controllers, micro-controllers, and microprocessors.

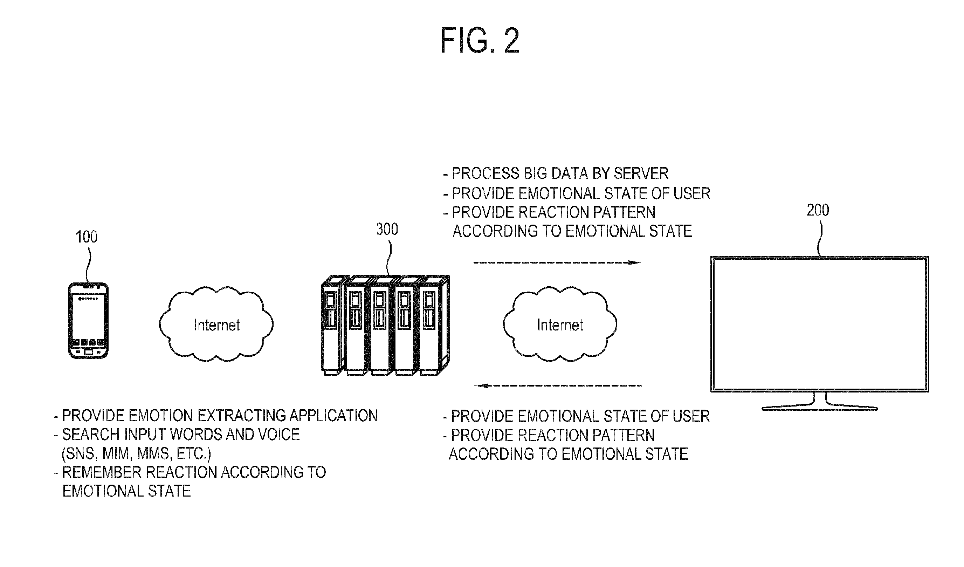

The aforementioned first electronic device 100 and second electronic device 200 which may determine emotions of the user may share the emotional state of the user as shown in FIG. 1, and may share the emotional state of the user in real-time by interposing the cloud server 300 therebetween for cloud service as shown in FIG. 2.

The first electronic device 100 and the second electronic device 200 which may determine the emotion of the user may include the storage units, respectively. The storage units may store therein, the input emotional data input through the user input unit, the reference emotional data to be compared with the input emotional data, the emotional state data of the user which is determined by comparing the input emotional data and the reference emotional data, and reaction record data of the user correlated with the emotional state data determined. As above, the first and second electronic devices 100 and 200 may directly exchange all data relating to the emotions of the user stored in the storage units.

Even if the first and second electronic devices 100 and 200 include the storage units storing therein the data relating to the emotions of the user, they may share the data through the cloud server 300.

Instead of (or in addition to) storing the data relating to the emotions of the user in the storage units, the first electronic device 100 and second electronic device 200 which may determine emotion of the user may store the data in the cloud server 300 and may receive the data from the cloud server 300 for usage.

The first and second electronic devices 100 and 200 may have an emotion determining application installed therein, and store a reaction according to the emotional state, e.g. at least one history of a functional controlling operation, a predetermined application program executing operation, and a predetermined content outputting operation, and may share the patterns through the wired/wireless communication units 120 and 220.

As shown in FIG. 2, if the cloud server 300 is interposed between the first and second electronic devices 100 and 200, the cloud server 300 may own big data corresponding to the emotional state of the user. The big data on the emotional state of the user stored in the cloud server 300 may be updated in real-time according to the emotional state of the user transmitted to all electronic devices connected to the cloud server 300.

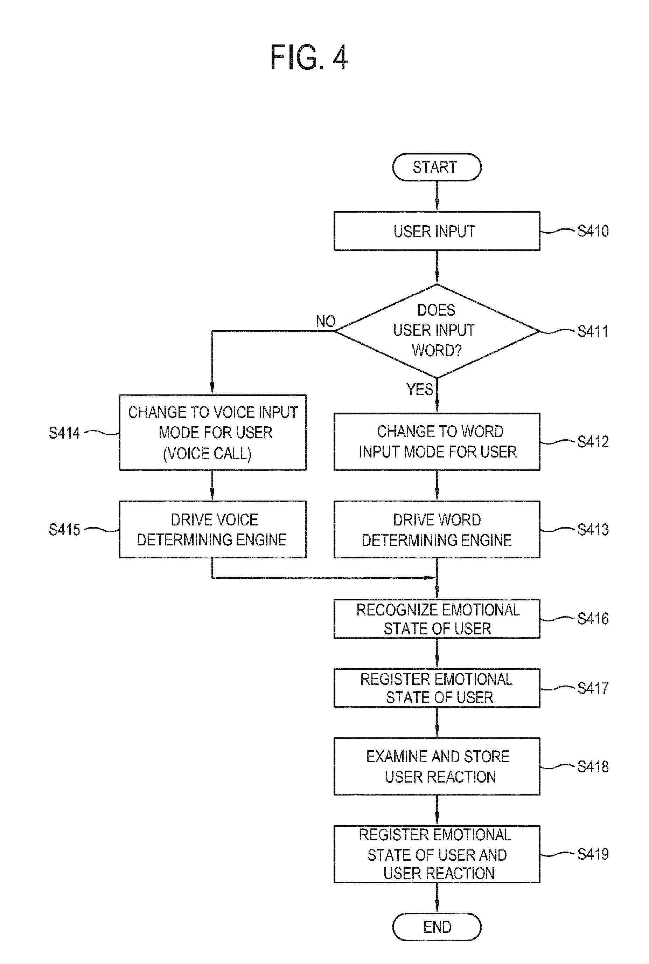

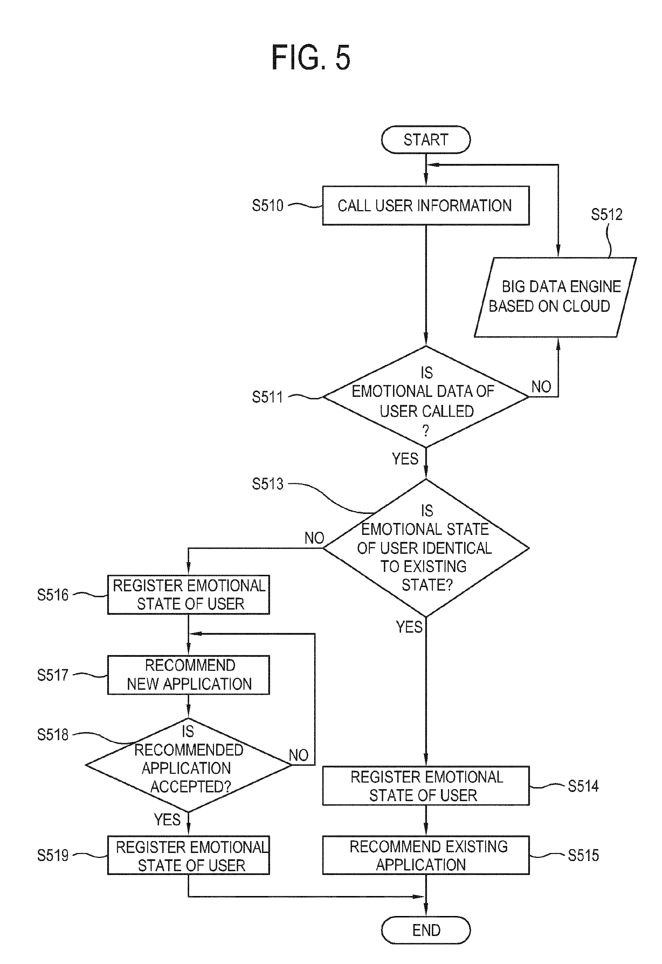

Hereinafter, a method for determining an emotion of the user using the electronic device according to an exemplary embodiment will be explained with reference to FIGS. 4 and 5.

FIG. 4 is a flowchart showing a method for determining an emotional state of the user through an emotion determining application and recording and registering a reaction according to the emotional state.

The user inputs the input emotional data including texts and/or voice with respect to chats or calls using an input device, e.g., a keyboard, a microphone, a remote controller, a camera (in a case a user using sign language that is converted to text and/or voice), etc., (S410).

It is determined whether the input emotional data input at operation S410 is text (S411). If the data includes a text, the electronic device is changed to the word input mode for the user (S412). Then, the word determining engine is driven to compare the input emotional data with the reference emotional data stored in the first storage unit 130 (S413).

If the data input at operation S410 is not a text but a voice, the electronic device is changed to the voice input mode for the user (S414). Then, the voice determining engine is driven to compare the input emotional data with the reference emotional data stored in the first storage unit 130 (S415). Typically, the emotion of the user may hardly be identified only through the voice itself. The user may speak loudly when the user is angry and may speak in a low voice when the user is depressed. However, measuring the emotional state of the user only with the voice may lack objectivity. If the voice of the user is particularly different from a normal calling pattern, such different voice may be used to identify the emotional state of the user.

Accordingly, the voice determining engine may identify the intention of the user through the voice recognition and compare the voice based on the intention rather than simply comparing the voices. Identifying the intention of the speaker requires an additional voice recognition database.

As described above, the word determining engine or voice determining engine may determine the emotional state of the user using the input emotional data of the user including the text or voice (S416). There may be a case where the emotional state of the user may not be determined using the word or voice determining engine even though the user input is made for considerable time. In this case, the input emotional data for determining emotion may not be present or there may be no word or voice matching with the reference emotional data.

The emotional state of the user determined at operation S416 is registered with the storage unit 130 (S417). If the emotional state of the user is determined as above, a user reaction made for predetermined time, i.e., the function controlling operation of the electronic device, the predetermined application program executing operation and the predetermined content outputting operation are identified and then the history is stored (S418). The functional controlling operation of the electronic device 100 may include input speed, rate of typographical error of texts, an intensity of touching a touch screen, power-off, repetition of channel change, speed and size of voice, rate of voice recognition, and other contents changing according to the emotional state. The predetermined application program executing operation may include an execution of a particular game program, execution of a music playing program, and execution of a video playing program. The predetermined content outputting operation may specify and record in detail types of content enjoyed by the user according to the emotional state of the user, e.g., action movies, comedies, melodramas, sports games, classic music, rock music, etc.

Lastly, the reaction pattern according to the emotional state of the user is correlated with the emotional state to be registered with the storage unit 130 (S419).

As above, the electronic device according to the exemplary embodiment may identify the emotional state of the user through the text or voice that is typically input by the user, and then recommend or suggest a reaction to the user according to the emotion of the user. The reaction may include a suggestion to the user using an electronic device for the user to consume a certain type of media based on the emotion of the user. The type of media suggested to the user can be to enhance the user's emotion, to change the user's emotion, etc.

FIG. 5 is a flowchart showing a method for receiving the emotional state of a single user at a TV 200 from a mobile phone 100 and responding to the emotional state of the user when the user watches the TV 200 while using the mobile phone 100.

If a user turns on the TV 200, the TV 200 registers a user's account, loads user information (S510), and checks whether the user information includes emotional state data of the user (S511). If there is no emotional state data of the user, the cloud-based big data engine is driven (S512) to load the user information again.

If there is emotional state data of the user, the emotional state is compared with the existing data to determine whether the emotional state and the emotional state data match (S513).

If the emotional state of the user matches to the existing data at operation S513, the emotional state of the user is registered (S514), and the existing application is recommended (S515).

If the emotional state of the user is different from the existing data at operation S513, the emotional state of the user is registered (S516), and a new application is recommended (S517).

The TV 200 checks whether the user accepts the recommended application (S518), and if the user does not accept the application, the TV 200 recommends another application.

If the user accepts the application at operation S518, the emotional state of the user is registered (S519).

As above, the method of determining the emotion of the user using the TV 200 with the mobile phone 100 based on the cloud service 300 and collecting the big data to provide feedback according to the emotional state of the viewer have been explained. That is, to determine the emotion of the user, the TV 200 and the mobile terminal 100 are provided with the emotion determining application. The application is configured to identify the emotional state of the user through two functions: (i) determining any word relating to emotions out of words or sentences input while the user uses the mobile terminal to determine the emotional state of the user primarily, and (ii) identifying high or low tone of the voice when a user is talking over the mobile terminal 100, through the voice recognition function of the mobile terminal 100 to determine the emotional state of the user.

The application remembers the user's emotional state, and stores the user's reaction while the user has the emotional state through the mobile terminal 100. If the user feels good, the user may listen to a cheerful music to maintain such emotional state or make such reaction as delivering good news to his/her friends and acquaintances through SNS. If the user is depressed, the user may listen to heavy music, or exercise and such reaction is stored. Based on such reactions, the application may create a reaction pattern according to the emotional state of the user.

If the same application is installed in a user's smart TV 200, the smart TV application may identify the user's emotional state in real-time by cooperating with the user's mobile terminal 100, or the user's emotional state determined by the mobile terminal 100 may be transmitted to the smart TV application. Then, the TV 200 may identify the emotional state of the user more clearly, and may transmit the reaction according to the emotional state of the user to the mobile terminal 100.

The user's emotional state may be classified into categories such as, for example, pleasure, sadness, depression (loneliness), annoyance, moderation, etc.

The electronic device enables the happy user to maintain his/her emotional state and remembers the reaction of the happy user. The electronic device recommends a sad user music, video and images healing the user. The reaction selected by the sad user is also stored. The electronic device attempts contact with acquaintances and friends who recently contacted the depressed (lonely) user, and helps the user to remember happy memories with them. The electronic device also recommends healing music, fun video and images to the depressed user. The electronic device recommends the annoyed user a calming music or meditative music and further provides breathing exercise used for yoga. The electronic device continuously identifies the emotional state of the moderate user and identifies any change of emotion. The electronic device recommends to a user having a moderate emotional state music that the user likes or attempts contact with friends or acquaintances who have not been contacted recently.

According to the exemplary embodiments, a user of a mobile terminal 100 such as a smart phone inputs texts or voice showing various emotions naturally while in use, and such input emotional data may be used to easily determine the emotion of the user.

Further, when the user of the mobile terminal uses another electronic device 200, the emotional state of the user and the reaction pattern determined by the mobile terminal 100 and be shared with the another electronic device, which may provide various reaction methods for the user.

Further, the emotion determining method is not commonly used with respect to all users but is correlated with each user's specific characteristics and may determine emotions more accurately.

In particular, the database for determining emotions of the user is cumulative and the emotions may be determined more accurately.

Also, the cloud server 300 may be used to share the emotional state of the user between various electronic devices. In particular, even the electronic devices which have limited user input means for expressing and inputting the emotional state of the user may identify the emotional state through a mobile phone having various emotional state input units and may be connected with the mobile phone and utilize the emotional state in real-time.

Although a few exemplary embodiments have been shown and described, it will be appreciated by those skilled in the art that changes may be made in these exemplary embodiments without departing from the principles of the invention, the range of which is defined in the appended claims.

## Claims

1. A method for determining an emotional state of a user of an electronic device, the method comprising:
receiving input emotional data which is input by the user of the electronic device;
comparing the input emotional data with reference emotional data and determining the emotional state of the user; and
transmitting the determined emotional state of the user to an external device to share the emotional state with the external device.

2. The method according to claim 1, wherein the reference emotional data are stored in at least one of a storage unit and a cloud server connected through a communication network.

3. The method according to claim 1 or 2, wherein the input emotional data are obtained from voice data input by the user.

4. The method according to any one of the preceding claims, wherein the input emotional data are obtained from text data input by the user.

5. The method according to claim 3, further comprising correlating the determined emotional state with at least one of intensity, speed, and rate of recognition of the input voice data and storing the correlated emotional state.

6. The method according to claim 4, further comprising:
correlating the determined emotional state with least one of an input speed, rate of typographical error of the text data input by the user, and intensity of touching a touch screen; and
storing the correlated emotional state.

7. The method according to any one of the preceding claims, further comprising:
correlating the determined emotional state with at least one of motion history of a functional control operation, a predetermined application executing operation, and a predetermined content outputting operation of the electronic device as a reaction operation according to the emotional state of the user for predetermined time after determining the emotional state; and
registering the correlated emotional state.

8. The method according to claim 7, further comprising recommending to the user, in response to the emotional state of the user being determined, at least one of a function of the electronic device, execution of application, and output of content, corresponding to the emotional state based on the registering.

9. The method according to any one of the preceding claims, further comprising storing the determined emotional state of the user in a storage unit or a cloud server connected through communication.

10. The method according to claim 7 or 8, wherein the motion history correlated with the emotional state is recorded in a storage unit or a cloud server connected through a communication network.

11. The method according to claim 7 or 8, wherein the motion history correlated with the emotional state is transmitted to an external device together with the emotional state of the user.

12. A method for controlling an electronic device according to an emotional state of a user, the method comprising:
receiving emotional state data of a user which is determined by an external device connected through a communication network;
checking a reaction record of the user corresponding to the emotional state of the user transmitted by the external device; and
controlling the electronic device according to the checked reaction of the user.

13. An electronic device for determining an emotional state of a user, the electronic device comprising:
a user input unit which receives input emotional data from a user;
an emotional determiner which compares the received input emotional data with reference emotional data and determines the emotional state of the user; and
a communication unit which transmits the determined emotional state of the user.

14. A method for controlling an electronic device by sharing an emotional state of a user, the method comprising:
receiving input emotional data which is input by a user and determining an emotional state of the user based on the input emotional data by a first electronic device, and transmitting the determined emotional state of the user to a second electronic device to share the determined emotional state of a user with the second electronic device; and
receiving the emotional state data of the user determined by the first electronic device connected to the second electronic device through a communication network, checking a reaction record of the user corresponding to the emotional state of the user transmitted by the external device and controlling the second device according to the checked reaction of the user.

15. A system for sharing an emotional state of a user between electronic devices, the system comprising:
a first electronic device which comprises a first user input unit to receive input emotional data from a user, a first emotional determiner to compare the received input emotional data with reference emotional data and to determine an emotional state of the user, and a first communication unit to transmit the determined emotional state of the user; and
a second electronic device which comprises a second communication unit to receive emotional state data of the user determined by the first electronic device and a controller to check a reaction record of the user corresponding to the received emotional state of the user from the first electronic device and perform a control operation according to the checked reaction of the user.
